# FASCICULE DE BREVET EUROPEEN

(11) **EP 2 687 506 B1**
(45) Date de publication et mention de la délivrance du brevet: **24.06.2015**
(21) Numéro de dépôt: 13176588.5
(22) Date de dépôt: 16.07.2013
(51) Int. Cl.: C07C 269/04, C12P 41/00, C07D 223/16

(54) **Procédé de synthèse enzymatique de la (7S)-1-(3,4-diméthoxy bicyclo[4.2.0]octa-1,3,5-triène 7-yl) N-méthyl méthanamine, et application à la synthèse de l'Ivabradine et de ses sels**
Enzymatisches Syntheseverfahren von (7S)-1-(3,4-Dimethoxybicyclo[4.2.0]octa-1,3,5-trien7-yl) N-methyl-methanamin, und Verwendung zur Synthese von Ivabradin und seinen Salzen
Method for enzymatic synthesis of (7S)-1-(3,4-dimethoxybicyclo[4.2.0]octa-1,3,5-triene-7-yl) N-methyl methanamine, and use for the synthesis of Ivabradine and the salts thereof

(30) Priorité: 17.07.2012 FR 1256913
(43) Date de publication de la demande: 22.01.2014
(73) Titulaire: Les Laboratoires Servier, 92284 Suresnes Cedex (FR)
(72) Inventeur: Pedragosa Moreau, Sandrine, 45100 ORLEANS (FR); Lefoulon, François, 45000 ORLEANS (FR); Moris Varas, Francisco, 33209 GIJON (ES); Gonzalez Sabin, Javier, 33012 OVIEDO (ES)

(56) Documents cités:
- EP-A1- 1 598 333
- EP-A1- 2 495 237
- WO-A1-2010/072409
- ORSAT B ET AL: "Homocarbonates as Substrates for the Enantioselective Enzymatic Protection of Amines", JOURNAL OF THE AMERICAN CHEMICAL SOCIETY, ACS PUBLICATIONS, US, vol. 118, no. 3, 1 janvier 1996 (1996-01-01), pages 712-713, XP002395195, ISSN: 0002-7863, DOI: 10.1021/JA9533048

## Description

La présente invention concerne un procédé de synthèse enzymatique du composé de formule (I), la (7S)-1-(3,4-diméthoxy bicyclo[4.2.0] octa-1,3,5-triène 7-yl) N-méthyl méthanamine : 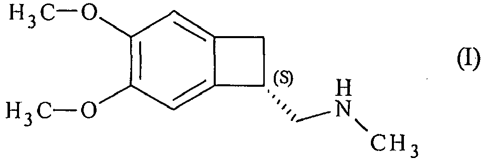 ainsi que son application à la synthèse de l'ivabradine de formule (II): 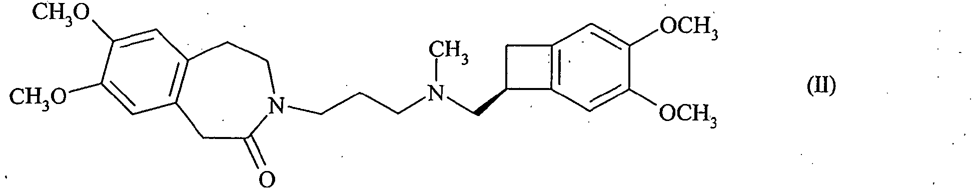 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (I).

Le composé de formule (I) est un intermédiaire-clé dans la synthèse de l'ivabradine et de ses sels pharmaceutiquement acceptables.

L'art antérieur divulgue plusieurs méthodes d'obtention du composé de formule (I).

Le brevet EP 0 534 859 décrit la synthèse du composé de formule (I) par réduction du nitrile racémique de formule (III): 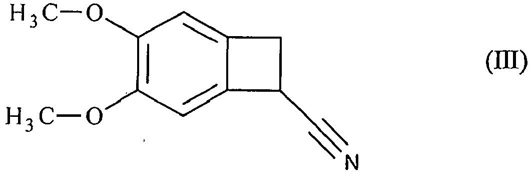 par BH₃ dans le tétrahydrofurane,
suivie de l'addition d'acide chlorhydrique, pour conduire au chlorhydrate de l'amine racémique de formule (IV) : 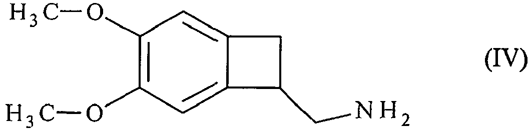 qui est mis en réaction avec du chloroformiate d'éthyle pour conduire au carbamate de formule (V) : 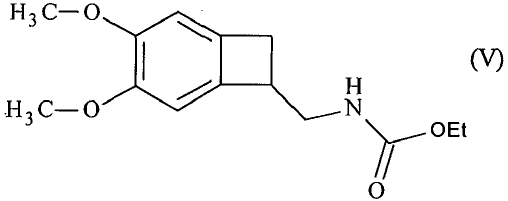 dont la réduction par LiAlH₄ conduit à l'amine méthylée racémique de formule (VI) : 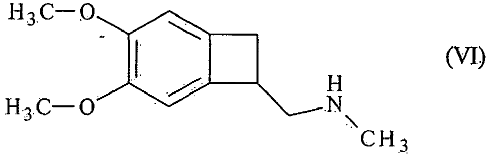 dont le dédoublement à l'aide d'acide camphosulfonique conduit au composé de formule (I). Cette méthode a l'inconvénient de ne conduire au composé de formule (I) qu'avec un rendement très faible de 2 à 3% à partir du nitrile racémique de formule (III).

Ce rendement très faible est dû au faible rendement (4 à 5%) de l'étape de dédoublement de l'amine secondaire de formule (VI).

La demande de brevet WO 2010/072409 décrit un procédé de résolution optique de la méthanamine de formule (VI). La publication J. Am. Chem. Soc 1996, 118, 712-713 décrit une méthode d'acylation enzymatique énantiosélective d'amines à l'aide de Subtilisin BPN' ou de lipase de *Candida rugosa.*

Le brevet EP 1 598 333 décrit l'obtention du composé de formule (I) par salification de l'amine primaire racémique de formule (IV) par l'acide N-acétyl-L-glutamique, suivie d'une recristallisation puis retour base, pour conduire à l'amine primaire optiquement active de formule (VII): 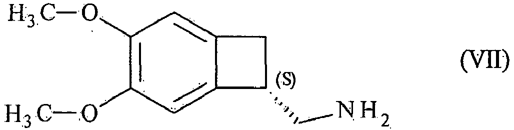 qui est ensuite méthylée par la même séquence réactionnelle que précédemment (transformation en carbamate, puis réduction).

Cette méthode conduit à la méthanamine de formule (I) en 4 étapes avec un rendement d'environ 30% à partir de l'amine primaire racémique de formule (IV).

Le problème de la présente invention était d'accéder au composé de formule (I) en réduisant le nombre d'étapes à partir de l'amine primaire racémique de formule (IV), tout en conservant un bon rendement global.

Plus spécifiquement, la présente invention concerne un procédé de synthèse du carbamate de formule (IX): 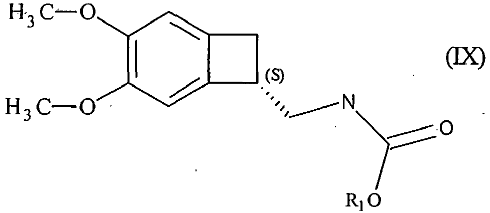 où R₁ représente un groupement alkyle C₁-C₆ linéaire ou ramifié, allyle ou benzyle,
par acylation enzymatique énantiosélective de l'amine racémique de formule (IV) à l'aide d'une lipase (EC 3.1.1.3 dans la classification internationale des enzymes),
par un carbonate de formule R₁O-(CO)-OR₁ où R₁ est tel que défini précédemment,
en quantité allant de 1 à 15 équivalents molaires par rapport à l'amine de formule (IV),
dans un solvant organique, aqueux, un mélange de solvants organiques ou un mélange de solvants organique et aqueux,
à une concentration de 5 à 500 g/L de composé de formule (IV) par litre de solvant ou mélange de solvants,
à un ratio E/S de 10/1 à 1/100, préférentiellement de 1/1 à 1/10,
à une température de 25°C à 40°C.

Le carbamate de formule (IX) obtenu selon le procédé de la présente invention a préférentiellement une pureté énantiomérique supérieure à 85%, soit un excès énantiomérique supérieur à 70%.

Parmi les lipases utilisables dans le procédé d'estérification enzymatique selon la présente invention, on peut citer à titre non limitatif les lipases de *Pseudomonas fluorescens,* de *Pseudomonas cepacia,* de *Pancreas porcine,* et les lipases PS 'Amano' SD (*Burkholderia cepacia*) et IM (immobilisée sur Diatomite).

Les lipases préférées selon l'invention sont les lipases de *Pseudomonas cepacia* et PS 'Amano' IM.

Les carbonates R₁O-(CO)-OR₁ préférés sont ceux pour lesquels R₁ représente un groupement allyle, éthyle ou benzyle.

Parmi les solvants organiques utilisables pour la réaction d'acylation enzymatique selon la présente invention, on peut citer à titre non limitatif l'acétate d'éthyle, le TBME, le THF, le 2-Me THF, le toluène, le 1,4-dioxane, le *tert*-amyl alcool, le CPME et l'acétonitrile.

Les solvants préférés sont le TBME, le THF, le 2-Me THF et le 1,4-dioxane, seuls ou en mélange avec un tampon à pH=7.

Le schéma de l'acylation enzymatique selon l'invention est le suivant :

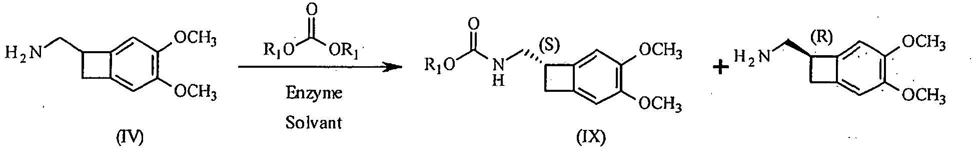

Le carbamate de formule (IX) est ensuite isolé du milieu réactionnel, puis il est réduit à l'aide d'un hydrure d'aluminium tel que l'hydrure de lithium aluminium LiAlH₄ ou l'hydrure de bis (2-méthoxy-éthoxy) sodium aluminium (RedAl),
pour conduire à l'amine méthylée de formule (I).

Celle-ci est ensuite couplée, soit avec un composé de formule (X) : 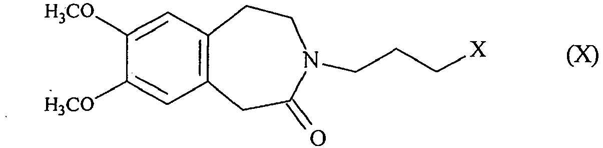 où X représente un atome d'halogène, préférentiellement un atome d'iode,
soit soumis à une réaction d'amination réductrice avec un composé de formule (XI) en présence d'un agent réducteur : 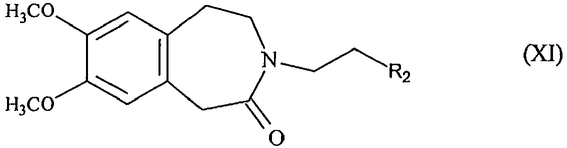 où R₂ représente un groupement choisi parmi CHO et CHR₃R₄,
où R₃ et R₄ représentent chacun un groupement alkoxy (C₁-C₆) linéaire ou ramifié, ou bien forment ensemble avec l'atome de carbone qui les porte un cycle 1,3-dioxane, 1,3-dioxolane ou 1,3-dioxépane,
pour conduire à l'ivabradine, qui est ensuite transformée en un sel d'addition à un acide pharmaceutiquement acceptable.

Le composé de formule (I) peut être également engagé dans la réaction d'amination réductrice sous la forme de son sel d'addition à un acide pharmaceutiquement acceptable, préférentiellement son chlorhydrate. Dans ce cas, l'ivabradine est obtenue directement sous la forme de chlorhydrate.

### Définitions

Par composé racémique, on entend composé sous la forme d'un mélange de deux énantiomères dans un rapport de 55:45 à 45:55.

Par acylation énantiosélective d'une amine sous la forme d'un mélange de deux énantiomères, on entend acylation préférentielle de l'un des énantiomères du mélange.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique.

Parmi les agents réducteurs utilisables pour la réaction d'amination réductrice entre le composé de formule (I) et le composé de formule (XI), on peut citer à titre non limitatif les composés donneurs d'hydrure tel que le triacétoxyborohydrure de sodium ou le cyanoborohydrure de sodium, et le dihydrogène en présence d'un catalyseur tel que le palladium, le platine, le nickel, le ruthénium, le rhodium ou un de leurs dérivés, notamment sous forme supportée ou sous forme d'oxydes.

L'agent réducteur préféré pour la réaction d'amination réductrice entre le composé de formule (I) et le composé de formule (XI) est le dihydrogène catalysé par le palladium sur charbon.

Les exemples ci-dessous illustrent l'invention.

### Abréviations

- CPME: CycloPentyl Méthyl Ether
- DEA: DiEthylAmine
- E: Coefficient d'énantiosélectivité
- E/S: ratio Enzyme/Substrat exprimé en g/g
- ee: excès énantiomérique
- éq: équivalent molaire
- HPLC: High Performance Liquid Chromatography (Chromatographie en phase liquide à haute performance)
- RedAl: Hydrure de bis (2-méthoxy-éthoxy) sodium Aluminium
- RMN: (Spectroscopie) Résonance Magnétique Nucléaire
- TBME: *Tert*-Butyl Méthyl Ether
- THF: TétraHydroFurane
- 2-Me THF: 2- Méthyl TétraHydroFurane
- tr/min: tours/min

### EXEMPLE 1 : {[(7S)-3,4-diméthoxybicyclo[4.2.0]octa-1,3,5-triène-7-yl]méthyl} carbamate d'éthyle

5 mg de 1-(3,4-diméthoxy bicyclo[4.2.0] octa-1,3,5-triène 7-yl) méthanamine et 12.6 mg (10 éq) de diéthylcarbonate sont solubilisés dans le 2-Me THF.

5 mg de lipase II de *Pseudomonas cepacia* (PS-CII Amano) sont alors ajoutés au milieu (ratio E/S 1/1). Le milieu réactionnel est maintenu à 30°C, sous agitation 250 tr/min rotative pendant 24h à 96h.

La réaction est contrôlée par HPLC sur phase chirale dans des conditions permettant de déterminer à la fois les excès énantiomériques du carbamate et de l'amine :

| | |
|---|---|
| *Conditions phase chirale :* | *colonne Chiralpak*® *IC 250*4.6* |
| | *50% éthanol absolu*+*0.1%DEA + 50%heptane+0.1%DEA 1ml.min 25°C 288nm* |

| **Concentration** | **t** | **Conversion c (%)** | **Ee (%) amine (R)** | **Ee (%) carbamate (S)** | **E** |
|---|---|---|---|---|---|
| 10 g/L | 24h | **38** | 57.7 | **92.3** | **45** |
| 20 g/L | 96h | **51** | **93.2** | **88.8** | **58** |
| 50 g/L | 96h | 57 | **99.9** | **76.1** | **69** |

### EXEMPLE 2 : {[(7S)-3,4-diméthoxybicyclo[4.2.0]octa-1,3,5-triène-7-yl]méthyl} carbamate d'éthyle

0.5 g de 1-(3,4-diméthoxy bicyclo[4.2.0] octa-1,3,5-triène 7-yl) méthanamine sont solubilisés dans 50 mL de 2 Me-THF puis on ajoute le carbonate de diéthyle (1.5mL, 12 éq). 0.5g (ratio E/S 1/1) de lipase II de *Pseudomonas cepacia* (PS-CII Amano) sont ajoutés au milieu qui est maintenu à 30°C pendant 48h sous agitation 220 tr/min.

Après 48h le milieu réactionnel est filtré pour éliminer l'enzyme puis évaporé. Le carbamate de configuration S est obtenu après séparation sur colonne de SiO₂ élution cyclohexane/acétate d'éthyle 95/5 puis 80/20 et enfin 50/50 pour récupérer l'amine plus polaire.

On obtient alors le carbamate d'éthyle de configuration S (224 mg) avec un rendement de 32.5 % par rapport à l'amine de départ (65% par rapport à la quantité attendue de carbamate) et une pureté énantiomérique de 90%.

La réaction est contrôlée par HPLC sur phase chirale dans des conditions permettant de déterminer à la fois les excès énantiomériques du carbamate et de l'amine :

| | |
|---|---|
| *Conditions phase chirale:* | *colonne Chiralpak*® *IC 250*4.6* |
| | *50% éthanol absolu+0.1%DEA + 50%heptane*+*0.1%DEA 1ml.min 25°C 288nm* |

| **Temps (h)** | **Conversion (%)** | **Ee (carbamate)(%)** | **Ee (amine) (%)** | **E** |
|---|---|---|---|---|
| 24 | 34 | 88 | 45 | 23 |
| 48 | 53 | 81 | 93 | 35 |

### EXEMPLE 3 : {[(7S)-3,4-diméthoxybicyclo[4.2.0]octa-1,3,5-triène-7-yl]méthyl} carbamate d'allyle

0.87 g de 1-(3,4-diméthoxy bicyclo[4.2.0] octa-1,3,5-triène 7-yl) méthanamine sont solubilisés dans 100 mL de 2 Me-THF puis on ajoute le carbonate de diallyle (1.5mL, ∼2éq). 0.5g (ratio E/S 1/1) de lipase II de *Pseudomonas cepacia* (PS-CII Amano) sont ajoutés au milieu qui est maintenu à 30° pendant 42h sous agitation à 220 tr/min.

Le milieu réactionnel est ensuite filtré pour éliminer l'enzyme puis évaporé. Le carbamate d'allyle est obtenu après séparation sur colonne de SiO₂ élution cyclohexane/acétate d'éthyle 95/5 puis 80/20 et enfin 50/50 pour récupérer l'amine plus polaire.

On obtient alors le carbamate d'allyle de configuration S (440 mg) avec un rendement de 35% par rapport à l'amine de départ (70% par rapport à la quantité attendue de carbamate) et une pureté énantiomérique de 88%.

| **Temps (h)** | **Conversion (%)** | **Ee (carbamate)(%)** | **Ee (amine) (%)** | **E** |
|---|---|---|---|---|
| 42 | 50 | 89 | 82 | 26 |

Le milieu réactionnel est analysé par HPLC en phase inverse et l'énantiosélectivité (ee) du carbamate et de l'amine sont contrôlées par HPLC en phase chirale selon les méthodes décrites ci-dessous :

| | | |
|---|---|---|
| *Conditions phase inverse :* | *colonne Phenomenex*® *LUNA HST 50*3* | *C18(2) 2.5µm* |
| | *0% à 100% de B en 8min 0.8m*/*.min* | *40°C* |
| | *A (1000 eau*+*25 ACN*+*1 ATFA)* | |
| | *B (1000 ACN+25 eau*+*1 ATFA)* | |
| | | |
| *Conditions phase chirale :* | *colonne Chiralpak*® *IC 250*4.6* | |
| | *50% isopropanol*+*0.1%AE* + *50%heptane*+*0.1%AE* | |
| | *1ml.min 30°C 288nm* | |

### EXEMPLE 4 : {[(7S)-3,4-diméthoxybicyclo[4.2.0]octa-1,3,5-triène-7-yl]méthyl} carbamate de benzyle

0.5 g de 1-(3,4-diméthoxy bicyclo[4.2.0] octa-1,3,5-triène 7-yl) méthanamine sont solubilisés dans 50 mL de 2 Me-THF puis on ajoute le carbonate de dibenzyle (4.5g, 7 éq)). 0.5g (ratio E/S 1/1) de lipase II de *Pseudomonas cepacia* (PS-C II Amano) sont ajoutés au milieu qui est maintenu à 30° sous agitation à 220 tr/min.

Après 24h, le milieu réactionnel est filtré pour éliminer l'enzyme puis évaporé. Le carbamate de configuration S est obtenu après séparation sur colonne de SiO₂ élution cyclohexane/acétate d'éthyle 95/5 puis 80/20 et enfin 50/50 pour récupérer l'amine plus polaire.

On obtient alors le carbamate de benzyle de configuration S (0.26 g) avec un rendement de 30% par rapport à l'amine de départ (60% par rapport à la quantité attendue de carbamate) et une pureté énantiomérique de 95%.

Le milieu réactionnel est analysé par HPLC en phase inverse et l'énantiosélectivité (ee) du carbamate et de l'amine sont contrôlées par HPLC en phase chirale selon les méthodes décrites ci-dessous :

| | | |
|---|---|---|
| *Conditions phase inverse :* | *colonne Phenomenex*® *LUNA HST 50*3* | *C18(2) 2.5µm* |
| | *0% à 100% de B en 8min 0.8ml.min* | *40°C* |
| | *A (1000 eau*+*25 ACN+1 ATFA)* | |
| | *B (1000 ACN*+*25 eau+1 ATFA*) | |
| | | |
| *Conditions phase chirale :* | *colonne Chiralpak*® *IC 250*4.6* | |
| | *50% isopropanol*+*0.1%DEA + 50%heptane+0.1%DEA* | |
| | *1ml.min 25°C 288nm* | |

| **Temps (h)** | **Conversion (%)** | **Ee (carbamate)(%)** | **Ee (amine) (%)** | **E** |
|---|---|---|---|---|
| 24 | 58 | 90 | 87 | 12 |

### EXEMPLE 5 : (7S)-3,4-Diméthoxybicyclo[4.2.0]octa-1,3,5-trién-7-yl]-N-méthyl-méthanamine

Dans un réacteur, charger sous azote l'hydrure de lithium et d'aluminium (1,41 kg), et du tétrahydrofurane (32,5 1), puis couler à 20°C une solution de {[(7*S*)-3,4-diméthoxybicyclo[4.2.0]octa-1,3,5-triène-7-yl]méthyl}carbamate d'éthyle (5 kg) dans le tétrahydrofurane (50 1). Chauffer au reflux pendant 1 heure, puis refroidir à une température inférieure à 15°C pour hydrolyser le mélange réactionnel par de l'eau (1 1), une solution aqueuse 5 N d'hydroxyde de sodium (11), puis de l'eau (11). Filtrer le solide obtenu. Mettre à sec la phase organique. On récupère le produit du titre sous la forme d'une huile, avec un rendement de 93%.

¹H RMN (DMSO-d6, ppm / TMS) = 2.60 (m; 3H); 2.85 (m; 1H); 3.15 (m; 1H); 3.25 (dd; 1H); 3.30 (m; 1H); 3.62 (m; 1H); 3.70 (s; 6H); 6.82 (s; 1H); 6.89 (s;1H); 8.48 (sl; 1H).

### EXEMPLE 6 : (7S)-3,4-Diméthoxybicyclo[4.2.0]octa-1,3,5-trién-7-yl]-N-méthyl-méthanamine, chlorhydrate

Dans un réacteur, charger la (7S)-3,4-diméthoxybicyclo[4.2.0]octa-1,3,5-trién-7-yl]-N-méthyl-méthanamine (5 kg), l'acétate d'éthyle (40 1) et l'éthanol (10 1). Agiter à 20°C pendant 30 mn, puis additionner par la vanne de fond du réacteur ou par un tube plongeant de l'acide chlorhydrique gazeux (1,012 kg). La suspension obtenue est agitée à 15-20°C pendant 1h, puis filtrée ou essorée. Le précipité est lavé par un mélange acétate d'éthyle/ éthanol 4/1 (2 x 5 1), puis séché, pour conduire au produit du titre avec un rendement de 92%.

### EXEMPLE 7 : Chlorhydrate de l'ivabradine

Dans un autoclave, charger 5.5 kg de 3-[2-(1,3-dioxolan-2-yl)éthyl]-7,8-diméthoxy-1,3-dihydro-2*H*-3-benzazépin-2-one, 27.5 l d'éthanol et 550 g de palladium sur charbon.

Purger à l'azote puis à l'hydrogène, chauffer à 55°C, puis hydrogéner à cette température sous une pression de 5 bars jusqu'à absorption de la quantité théorique d'hydrogène.

Ramener ensuite à température ambiante, puis décompresser l'autoclave.

Ajouter ensuite 4 kg du chlorhydrate de la (7S)-3,4-diméthoxybicyclo[4.2.0]octa-1,3,5-trién-7-yl]-N-méthylméthanamine, 11 l d'éthanol, 5.5 l d'eau et 1 kg de palladium sur charbon. Purger à l'azote puis à l'hydrogène, chauffer à 85°C, puis hydrogéner à cette température sous une pression de 30 bars jusqu'à absorption de la quantité théorique d'hydrogène.

Revenir ensuite à température ambiante, purger l'autoclave, puis filtrer le mélange réactionnel, distiller les solvants puis isoler le chlorhydrate d'ivabradine par cristallisation dans un mélange toluène/1-méthyl-2-pyrrolidinone.

Le chlorhydrate d'ivabradine est ainsi obtenu avec un rendement de 85 % et une pureté chimique supérieure à 99 %.

### EXEMPLE 8 : Screening de lipases pour l'acylation enzymatique de la 1-(3,4-diméthoxy bicyclo[4.2.0] octa-1,3,5-triène 7-yl) méthanamine

La 1-(3,4-diméthoxy bicyclo[4.2.0] octa-1,3,5-triène 7-yl) méthanamine racémique (5 mg; c=10 g/L) et le carbonate de formule R₁O-(CO)-OR₁ (10 éq) sont solubilisés dans 0.5 mL de TBME.

5 mg (c=10 g/L) de la lipase étudiée sont alors ajoutés au milieu (ratio E/S=1/1). Le milieu réactionnel est maintenu à 30°C, sous agitation 250 tr/min rotative pendant 24h.

Les milieux réactionnels sont analysés par HPLC en phase chirale pour le contrôle de l'énantiosélectivité selon la méthode :
*colonne Chiralpak® IC 20 um, 250*4.6 Acétonitrile*/*propan-2-ol*/*DEA 90*/*10*/*0.1%; 1.3ml.min ; 30°C 288nm*

Les résultats sont résumés dans le tableau suivant :

| **Lipase** | **Carbonate** | **Produit** | ***Conversion c* (*%*)** | ***ee* (*%*) Amine (R)** | ***ee* (*%*) Carbamate (S)** | ***E*** |
|---|---|---|---|---|---|---|
| *Pseudomonas cepacia* lipase II | 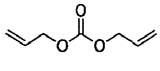 | **IXa** | 59 | >99.9 | 69.8 | 40 |
| *Pseudomonas fluorescens* | | | 14 | 12.3 | 73.8 | 7 |
| Lipase PS 'Amano' SD | | | 4 | 3.9 | 83.2 | 11 |
| Lipase PS 'Amano' IM | | | 52 | 91.6 | 83.5 | 36 |
| *Pseudomonas cepacia* lipase II | 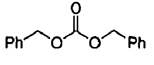 | **IXb** | 57 | 97.0 | 73.4 | 26 |
| *Pseudomonas fluorescens* | | | 5 | 3.9 | 78.2 | 8 |
| Lipase PS 'Amano' IM | | | 33 | 44.4 | 89.0 | 27 |
| *Pseudomonas cepacia* lipase II | 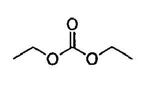 | **IXc** | 16 | 17.7 | 89.6 | 22 |
| *Pseudomonas fluorescens* | | | 3 | 2.2 | 66.1 | 5 |
| Lipase PS 'Amano' IM | | | 12 | 11.2 | 84.6 | 13 |
| 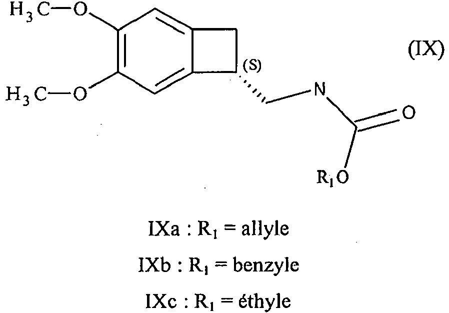 | | | | | | |

| | | | | | | |
|---|---|---|---|---|---|---|
| ^{a} Excès énantiomérique ee (en %) = % énantioE2 - % énantioE1 / % énantio E2 + % énantio E1 (énantio E2 étant l'énantiomère majoritaire) ^{b} coefficient d'énantiosélectivité E = ln[(1-c)(1-ee(S)] / ln[(1-c)(1+ee(S)] ; c= taux de conversion = ee(amine) /[ee(carbamate) + ee(amine)] | | | | | | |

## Revendications

1. Procédé de synthèse du composé de formule (IX): 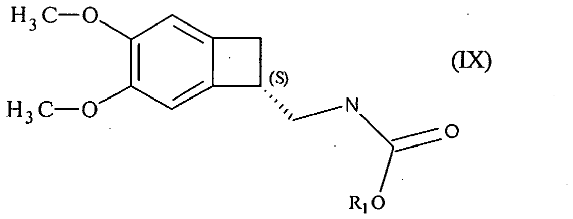 où R₁ représente un groupement alkyle C₁-C₆ linéaire ou ramifié, allyle ou benzyle,
par acylation enzymatique énantiosélective de l'amine racémique de formule (IV) : 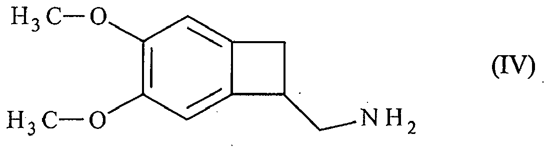 à l'aide d'une lipase (EC 3.1.1.3 dans la classification internationale des enzymes) :
par un carbonate de formule R₁O-(CO)-OR₁ où R₁ est tel que défini précédemment,
en quantité allant de 1 à 15 équivalents molaires par rapport à l'amine de formule (IV), dans un solvant organique, aqueux, un mélange de solvants organiques ou un mélange de solvants organique et aqueux,
à une concentration de 5 à 500 g/L de composé de formule (IV) par litre de solvant ou mélange de solvants,
à un ratio E/S de 10/1 à 1/100, à une température de 25°C à 40°C.

2. Procédé de synthèse selon la revendication 1, dans lequel la lipase est choisie parmi les lipases de *Pseudomonas fluorescens,* de *Pseudomonas cepacia,* de *Pancreas porcine,* et les lipases PS 'Amano' SD (*Burkholderia cepacia*) et IM (immobilisée sur Diatomite).

3. Procédé de synthèse selon la revendication 2, dans lequel la lipase est une lipase de *Pseudomonas cepacia* ou une lipase PS 'Amano' IM.

4. Procédé de synthèse selon l'une quelconque des revendications 1 à 3, dans lequel le ratio E/S est de 1/1 à 1/10.

5. Procédé de synthèse selon l'une quelconque des revendications 1 à 4, dans lequel le solvant est choisi parmi le TBME, le THF, le 2-Me THF et le 1,4-dioxane, seul ou en mélange avec un tampon à pH=7.

6. Procédé de synthèse selon l'une quelconque des revendications 1 à 5, dans lequel R₁ est un groupement éthyle, allyle ou benzyle.

7. Procédé de synthèse du composé de formule (I) : 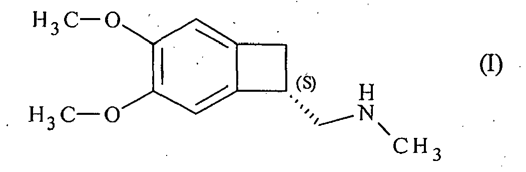 par acylation enzymatique de l'amine racémique de formule (IV) selon l'une quelconque des revendications 1 à 6, pour conduire au carbamate de formule (IX) : 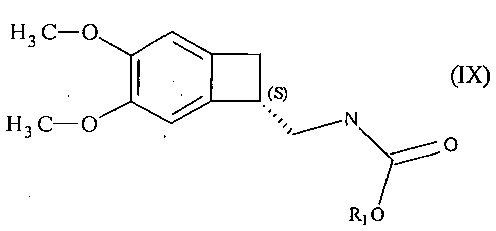 où R₁ représente un groupement alkyle C₁-C₆ linéaire ou ramifié, allyle ou benzyle,
qui est ensuite réduit par un agent réducteur choisi parmi LiAlH₄ et RedAl, pour conduire au composé de formule (I).

8. Procédé de synthèse selon la revendication 7, dans lequel le composé de formule (I) est ensuite soit couplé avec un composé de formule (X) : 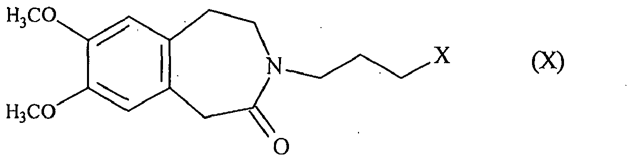 où X représente un atome d'halogène,
soit soumis à une réaction d'amination réductrice avec un composé de formule (XI) en présence d'un agent réducteur : 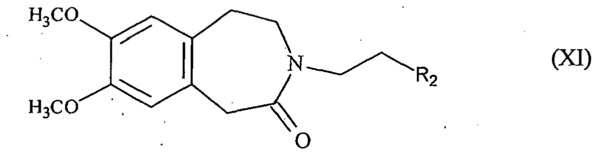 où R₂ représente un groupement choisi parmi CHO et CHR₃R₄,
où R₃ et R₄ représentent chacun un groupement alkoxy (C₁-C₆) linéaire ou ramifié, ou bien forment ensemble avec l'atome de carbone qui les porte un cycle 1,3-dioxane, 1,3-dioxolane ou 1,3-dioxépane,
pour conduire à l'ivabradine, qui est ensuite transformée en un sel d'addition à un acide pharmaceutiquement acceptable sous forme anhydre ou hydrate.

9. Procédé de synthèse selon la revendication 8, dans lequel X est un atome d'iode.

10. Procédé de synthèse selon la revendication 8, dans lequel le composé de formule (I) est engagé dans la réaction d'amination réductrice sous la forme de son chlorhydrate, pour conduire à l'ivabradine sous la forme de chlorhydrate.

11. Procédé de synthèse selon l'une quelconque des revendications 8 ou 10, dans lequel la réaction d'amination réductrice avec un composé de formule (XI) est effectuée en présence de dihydrogène catalysé par le palladium sur charbon.

## Patentansprüche

1. Verfahren zur Synthese der Verbindung der Formel (IX): 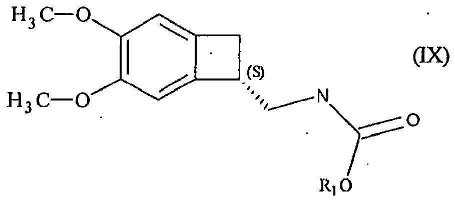 worin R₁ eine geradkettige oder verzweigte C₁-C₆-Alkylgruppe, Allylgruppe oder Benzylgruppe bedeutet,
durch enantioselektive enzymatische Acylierung des racemischen Amins der Formel (IV): 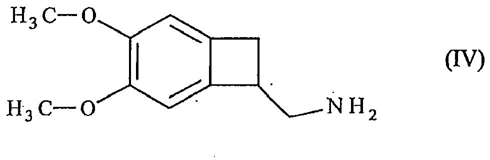 mit Hilfe einer Lipase (EC 3.1.1.3 nach der internationalen Klassifizierung der Enzyme):
mit einem Carbonat der Formel R₁O-(CO)-OR₁, worin R₁ die oben angegebenen Bedeutungen besitzt,
in einer Menge von 1 bis 15 Mol-Äquivalenten bezogen auf das Amin der Formel (IV), in einem organischen Lösungsmittel, wässrigen Lösungsmittel, einer Mischung von organischen Lösungsmitteln oder eine Mischung von organischen und wässrigen Lösungsmitteln,
bei einer Konzentration von 5 bis 500 g/l der Verbindung der Formel (IV) pro Liter des Lösungsmittels oder der Lösungsmittelmischung,
bei einem E/S-Verhältnis von 10/1 bis 1/100 bei einer Temperatur von 25 °C bis 40 °C.

2. Syntheseverfahren nach Anspruch 1, worin die Lipase aus Lipasen von *Pseudomonas fluorescens, Pseudomonas cepacia, Pancreas porcine* und die Lipasen PS 'Amano' SD (*Burkholderia cepacia*) und IM (auf Diatomit immobilisiert) ausgewählt wird.

3. Syntheseverfahren nach Anspruch 2, worin die Lipase eine Lipase von *Pseudomonas cepacia* oder eine Lipase PS 'Amano' IM ist.

4. Syntheseverfahren nach einem der Ansprüche 1 bis 3, worin das E/S-Verhältnis 1/1 bis 1/10 beträgt.

5. Syntheseverfahren nach einem der Ansprüche 1 bis 4, worin das Lösungsmittel aus TBME, THF, 2-Me THF und 1,4-Dioxan allein oder in Mischung mit einem Puffer mit einem pH-Wert von 7 ausgewählt wird.

6. Syntheseverfahren nach einem der Ansprüche 1 bis 5, worin R₁ eine Ethyl-, Allyl- oder Benzylgruppe ist.

7. Verfahren zur Synthese der Verbindung der Formel (I): 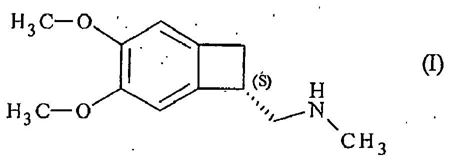 durch enzymatische Acylierung des racemischen Amins der Formel (IV) nach einem der Ansprüche 1 bis 6 zur Bildung des Carbamats der Formel (IX): 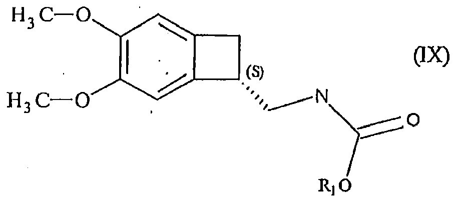 worin R₁ eine geradkettige oder verzweigte C₁-C₆-Alkylgruppe, Allylgruppe oder Benzylgruppe bedeutet,
welches anschließend mit einem Reduktionsmittel ausgewählt aus LiAlH₄ und RedAl reduziert wird zur Bildung der Verbindung der Formel (I).

8. Syntheseverfahren nach Anspruch 7, worin die Verbindung der Formel (I) anschließend mit einer Verbindung der Formel (X) gekuppelt wird: 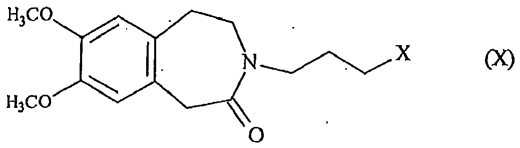 worin X ein Halogenatom bedeutet,
und anschließend einer reduzierenden Aminierungsreaktion mit einer Verbindung der Formel (XI) in Gegenwart eines Reduktionsmittels unterworfen wird: 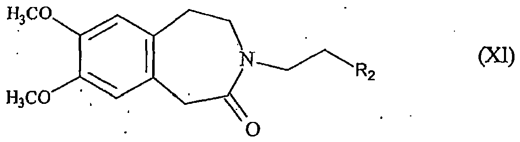 worin R₂ eine Gruppe ausgewählt aus CHO und CHR₃R₄ bedeutet,
worin R₃ und R₄ jeweils eine geradkettige oder verzweigte (C₁-C₆)-Alkoxygruppe bedeuten oder gemeinsam mit dem sie tragenden Kohlenstoffatom einen 1,3-Dioxan-, 1,3-Dioxolan- oder 1,3-Dioxepanring bilden,
zur Bildung von Ivabradin, welches anschließend in ein Additionssalz mit einer pharmazeutisch annehmbaren Säure in wasserfreier Form oder eines Hydrats umgewandelt wird.

9. Syntheseverfahren nach Anspruch 8, worin X ein Iodatom bedeutet.

10. Syntheseverfahren nach Anspruch 8, worin die Verbindung der Formel (I) in Form ihres Hydrochlorids bei der reduzierenden Aminierungsreaktion eingesetzt wird zur Bildung von Ivabradin in Form des Hydrochlorids.

11. Syntheseverfahren nach einem der Ansprüche 8 oder 10, worin die reduzierende Aminierungsreaktion mit der Verbindung der Formel (XI) in Gegenwart von Wasserstoff und Palladium-auf-Kohlenstoff als Katalysator durchgeführt wird.

## Claims

1. Process for the synthesis of the compound of formula (IX): 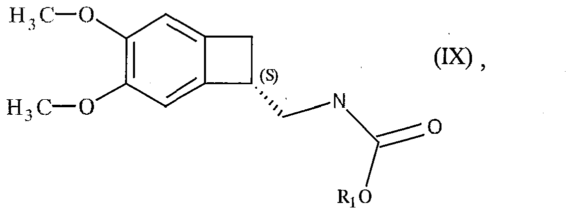 wherein R₁ represents a linear or branched C₁-C₆alkyl, allyl or benzyl group,
by enantioselective enzymatic acylation of the racemic amine of formula (IV): 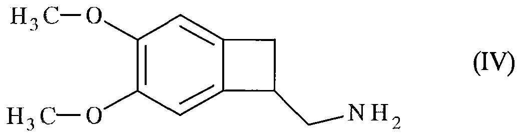 using a lipase (EC 3.1.1.3 in the international classification of enzymes),
with a carbonate of formula R₁O-(CO)-OR₁, wherein R₁ is as defined hereinbefore,
in an amount ranging from 1 to 15 molar equivalents relative to the amine of formula (IV),
in an organic or aqueous solvent, a mixture of organic solvents or a mixture of organic and aqueous solvents,
at a concentration from 5 to 500 g/L of compound of formula (IV) per litre of solvent or mixture of solvents,
at an E/S ratio of from 10/1 to 1/100, at a temperature from 25°C to 40°C.

2. Synthesis process according to claim 1, wherein the lipase is selected from lipases of *Pseudomonas fluorescens,* of *Pseudomonas cepacia,* of porcine pancreas, and the lipases PS 'Amano' SD (*Burkholderia cepacia*) and IM (immobilised on Diatomite).

3. Synthesis process according to claim 2, wherein the lipase is a lipase of *Pseudomonas cepacia* or a lipase PS 'Amano' IM.

4. Synthesis process according to any one of claims 1 to 3, wherein the E/S ratio is from 1/1 to 1/10.

5. Synthesis process according to any one of claims 1 to 4, wherein the solvent is selected from TBME, THF, 2-MeTHF and 1,4-dioxane, on their own or in admixture with a buffer of pH=7.

6. Synthesis process according to any one of claims 1 to 5, wherein R₁ is an ethyl, allyl or benzyl group.

7. Process for the synthesis of the compound of formula (I): 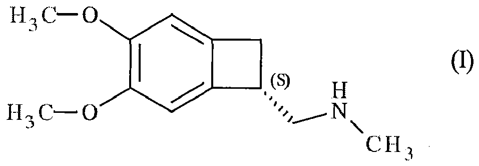 by enzymatic acylation of the racemic amine of formula (IV) in accordance with any one of claims 1 to 6 to yield the carbamate of formula (IX): 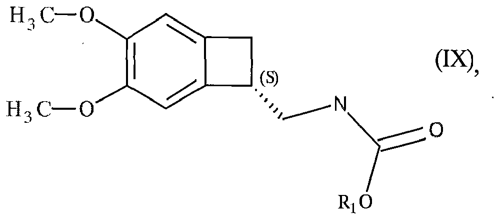 wherein R₁ represents a linear or branched C₁-C₆alkyl, allyl or benzyl group,
which is then reduced using a reducing agent selected from LiAlH₄ and Red-Al to yield the compound of formula (I).

8. Synthesis process according to claim 7, wherein the compound of formula (I) subsequently is either coupled with a compound of formula (X): 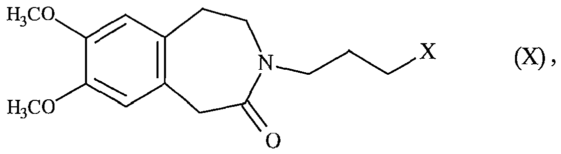 wherein X represents a halogen atom,
or, in the presence of a reducing agent, subjected to a reductive amination reaction with a compound of formula (XI): 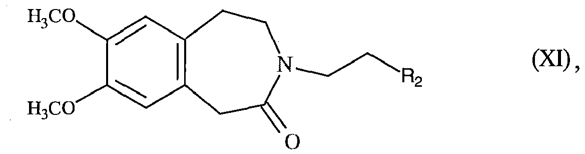 wherein R₂ represents a group selected from CHO and CHR₃R₄,
wherein R₃ and R₄ each represent a linear or branched (C₁-C₆)alkoxy group or form, together with the carbon atom carrying them, a 1,3-dioxane, 1,3-dioxolane or 1,3-dioxepane ring,
to yield ivabradine, which is then converted into an addition salt with a pharmaceutically acceptable acid, in anhydrous or hydrate form.

9. Synthesis process according to claim 8, wherein X is an iodine atom.

10. Synthesis process according to claim 8, wherein the compound of formula (I) is used in the form of its hydrochloride in the reductive amination reaction, to yield ivabradine in the form of the hydrochloride.

11. Synthesis process according to either claim 8 or claim 10, wherein the reductive amination reaction with a compound of formula (XI) is carried out in the presence of dihydrogen catalysed by palladium-on-carbon.
